# EUROPÄISCHE PATENTSCHRIFT

(11) **EP 3 723 339 B1**
(45) Veröffentlichungstag und Bekanntmachung des Hinweises auf die Patenterteilung: **14.09.2022**
(21) Anmeldenummer: 20168676.3
(22) Anmeldetag: 08.04.2020
(51) Int. Cl.: H04W 12/08, H04W 12/00, G16H 10/60, G16H 10/65, H04W 12/084, H04W 12/61, H04L 9/40, G06F 21/62

(54) **SICHERE FREIGABE EINER GESCHÜTZTEN FUNKTION**
SECURE RELEASE OF PROTECTED FUNCTION
LIBÉRATION SÉCURISÉE D'UNE FONCTION PROTÉGÉE

(30) Priorität: 08.04.2019 EP 19167932
(43) Veröffentlichungstag der Anmeldung: 14.10.2020
(73) Patentinhaber: myneva Group GmbH, 22309 Hamburg (DE)
(72) Erfinder: HOFMEISTER, Helge, 20149 Hamburg (DE)
(74) Vertreter: Raffay & Fleck

(56) Entgegenhaltungen:
- WO-A1-2016/033208
- US-A1- 2015 213 195
- US-A1- 2016 117 448

## Beschreibung

Die vorliegende Erfindung betrifft ein Verfahren zur Freigabe einer geschützten Funktion, insbesondere Ausgabe mindestens einer geschützten Information.

Informationen und Daten werden heutzutage an unterschiedlichen Orten gespeichert und sollen gegebenenfalls von überall mittels unterschiedlicher Methoden und Funktionen abrufbar sein. Allerdings soll eine solche Funktion, die zum Beispiel die Information anzeigt, nicht ohne Weiteres oder für jeden ausführbar bzw. abrufbar sein. Auch andere Funktionen sollen teilweise nicht beliebig ausführbar sein, sondern insbesondere vor unbefugtem Zugriff geschützt werden.

Um den Zugriff auf die Funktion, somit zum Beispiel den Zugriff auf die Funktion, die die Informationen freigibt oder anzeigt, zu beschränken, ist bekannt, den Zugang zu der Funktion oder die Funktion selbst zu sichern oder zu steuern. Hierzu ist vor allem bekannt, dass der Zugang durch ein Passwort oder andere Sicherheitsmaßnahmen gesichert wird. Auch eine Sicherung mittels mehreren, unter Umständen auch aufeinander aufbauenden, Sicherungsmaßnahmen ist bekannt, wie z. B. die sogenannten Zwei-Faktor-Authentisierung (2FA). Ein solches System ist beispielsweise aus der US 2016/0117448 A1 bekannt. In dieser wird zudem vorgeschlagen, dass neben einem Benutzer, also einer Person, die zur Freigabe berechtigt ist und die Freigabe der Information veranlassen darf, auch eine weitere von dieser Person vorausgewählte Person oder ein vorbestimmter Nutzer die Freigabe jeweils nach Ablauf einer Wartezeit veranlassen dürfen. Bei dem Nutzer handelt es sich dabei um einen berechtigten und zuvor zertifizierten Nutzer, nämlich den Gesundheitsdienstleister, der von dem System der US 201 6/0117448 A1 hierfür zuvor zertifiziert wurde. Beispielsweise erfolgt die Freigabe im Falle des Gesundheitsdienstleister infolge einer den Autorisierungsvorgangs überschreibenden (teilweise) bestätigende Rückmeldung (sogenannte "Break-The-Glass-Procedure" oder "Break-The-Glass-Option").

Funktionen bzw. Informationen, die geschützt werden, sind unter anderem Bankdaten sowie Bankfunktionen, Webseiteninhalte sowie Webseitenzugriffe oder Daten sowie Datenzugriffe. Ein System, welches den Zugang zu Webseiten reguliert und steuert, ist beispielsweise in der US 9,253,174 B1 dargelegt, bei welchem zum Beispiel Kindern der Zugang zu bestimmten Webseiten durch Sicherungsregeln verweigert wird, wobei diese Sicherungsregeln eine vorher bestimmte Liste und eine zweite Zustimmung der Freigabe des Zugriffs auf die in der Liste gelisteten Webseite umfasst. Somit kann zum Beispiel der Zugriff auf bestimmte Webseiten durch die Eltern reguliert werden, ohne den Zugriff auf den Inhalt des Internets komplett blocken zu müssen.

Auch bekannt sind System und Verfahren, die den Zugriff auf medizinische bzw. klinische Daten und System sichern und beschränken. Hierzu gibt die US 2008/0104410 A1 ein System und Verfahren an, bei welchem nach einer Eingabe eines alphanumerischen Passworts durch einen Benutzer ein physischer personalisierter Identifizierer des Users als zweite Sicherheitsschranke eingebaut wurde und überwunden werden muss, um die gesicherte Information bzw. die beschränkte Funktion abrufen bzw. nutzen zu könne. US 2016/117448 A1 offenbart ein Verfahren zum Auffüllen eines ausschließlich vom Patienten kontrollierten Datensatzes in einem vom Patienten kontrollierten elektronischen Gesundheitsdatenspeicher, das die Verarbeitung einer Anfrage nach einem Gesundheitsinformationsartefakt und das Senden eines verschlüsselten Informationsanforderungsdokuments beinhaltet. Die Erfindung wird durch die angehängten unabhängigen Ansprüche definiert.

Die Erfinder haben erkannt, dass bezüglich der derzeitigen Sicherungsmaßnahmen unter bestimmten Voraussetzungen die bisher bekannten Systeme und Vorrichtungen zu restriktiv gestaltet sein können, und haben dieses Problem mithilfe des vorliegenden Verfahrens nach Anspruch 1 sowie einem System nach Anspruch 14 gelöst. Vorteilhafte Weiterbildungen des Verfahrens und des Systems sind in den Ansprüchen 2 bis 13 gegeben.

Beispielsweise ist das erfindungsgemäße Verfahren geeignet, die Freigabe von Gesundheitsdaten einer Person zu regeln. Die Person kann beispielsweise einen QR Code mitführen, in dem besonders wichtige Gesundheitsdaten verschlüsselt und eine ID codiert sind. Der Notarzt kann dann mit einem Lesegerät den QR Code auslesen und die darin verschlüsselt Gesundheitsdaten entschlüsseln und mittels der ID bei einem Server weitere Gesundheitsdaten anfragen. Dies kann eine Berechtigungsanfrage auf dem Smartphone der Person auslösen. Ist die Person bewusstlos und erfolgt somit keine Antwort auf die Berechtigungsanfrage, wird dem Notarzt eine erste Information angezeigt.

In einer anderen Situation, in der die Person eine Arztpraxis aufsucht und ihren QR Code scannen lässt, kann die Arztpraxis mittels der ID bei einem Server weitere Gesundheitsdaten anfragen. Dies kann wieder eine Berechtigungsanfrage auf dem Smartphone der Person auslösen. Diese kann nun angeben, welche Gesundheitsdaten freigegeben werden sollen und eine entsprechende Rückmeldung an den Server absetzen, auf die hin dann die ausgewählten Gesundheitsdaten an die Arztpraxis übermittelt werden.

Wird der QR Code beispielsweise von einer unberechtigten Person erfasst und eine Anfrage an den Server gesendet, kann die Person die Berechtigungsanfrage beispielswiese ablehnen, sodass keine Gesundheitsdaten angefragt werden.

Die Erfinder haben erkannt, dass es in manchen Fällen, wie oben beschrieben, sinnvoll ist, auch bei Ausbleiben einer Bestätigung oder Ablehnung, ein solches "Schweigen" nicht als Ablehnung zu werten werden darf, aber ebenso wenig als Bestätigung. Aufgrund dessen wird in dem erfindungsgemäßen Verfahren bei einem solchen Ausbleiben einer, insbesondere jeder, Rückmeldung hinsichtlich des Autorisierungsvorgangs eine geschützte Funktion freigegeben, insbesondere eine geschützte Information ausgegeben, im weiteren Verlauf der Anmeldung erste Freigabe genannt. Diese erste Freigabe dieser geschützten Funktion/Information erfolgt beispielsweise auf dem Ausgabegerät. Bei einer Bestätigung kann eine andere oder umfassendere geschützte Funktion freigegeben, insbesondere eine andere oder umfassendere geschützte Information ausgegeben werden, im weiteren Verlauf der Anmeldung zweite Freigabe genannt. Das erfindungsgemäße Verfahren umfasst die Freigabe mindestens einer geschützten Funktion. Insbesondere umfasst das erfindungsgemäße Verfahren die Ausgabe mindestens einer geschützten Information. Eine geschützte Funktion kann beispielsweise eine Ausgabe mindestens einer geschützten Information oder die Funktionen eines technischen und/oder (halb-)automatischen Gerätes, insbesondere eines Automaten, wie beispielsweise eines Bankautomats, Lebensmittelautomats oder Fertigungsroboters, umfassen. Insbesondere kann die Freigabe auch eine Aktivierung einer Funktion darstellen.

Unter geschützte Funktion und/oder Information ist insbesondere jegliche Funktion und/oder Information zu verstehen, zu welchen Unberechtigte nicht ohne Weiteres Zugang haben bzw. welche Unberechtigte nicht ohne Weiteres ausführen können und/oder welche mittels einer Sicherung, wie beispielsweise einer Verschlüsselung und/oder Passwortsicherung, vor unberechtigten Zugriff geschützt sind. Vorteilhafterweise kann es sich bei der geschützten Funktion um eine Mehrzahl von geschützten Funktionen, insbesondere bei der geschützten Information um eine Mehrzahl geschützter Information, handeln. Vorteilhafterweise kann die Mehrzahl von geschützten Funktion zumindest eine erste geschützte Funktion, insbesondere erste Mehrzahl von geschützten Funktionen, und eine zweite geschützte Funktion, insbesondere zweite Mehrzahl von geschützten Funktionen, beinhalten. Vorteilhafterweise kann die Mehrzahl von geschützten Informationen zumindest eine erste geschützte Information, insbesondere erste Mehrzahl von geschützten Informationen, und eine zweite geschützte Information, insbesondere zweite Mehrzahl von geschützten Informationen, beinhalten. Vorteilhafterweise ist die erste geschützte Funktion, insbesondere erste Mehrzahl geschützter Funktionen, nicht identisch mit der zweiten geschützten Funktion, insbesondere ist die erste geschützte Information, insbesondere erste Mehrzahl geschützter Informationen, nicht identisch mit der zweiten geschützten Information, insbesondere erste Mehrzahl geschützter Informationen. Insbesondere beinhaltet die zweite Information und/oder die zweite Funktion die erste Information und/oder erste Funktion und insbesondere darüber hinaus weitere Information und/oder Funktion.

Das erfindungsgemäße Verfahren beinhaltet zunächst das Durchführen einer ersten Erfassung. Diese Erfassung erfolgt insbesondere mittels eines Erfassungsgeräts und/oder Eingabegeräts. Eine Erfassung ist beispielsweise mittels eines Erfassens einer Eingabe oder einer Information gegeben, wobei vorteilhafterweise die Eingabe ein Eingeben und/oder Einlesen einer Information, insbesondere in ein Eingabegerät, oder das Erfassen einer Information, wie z. B. die Anwesenheit einer Person oder eines Gerätes, ist. Insbesondere wird die erste Erfassung durch Eingabe eines Codes und/oder durch Scannen oder Auslesen eines Codes durchgeführt, beispielsweise durch Fotografieren eines Barcodes oder durch Auslesen einer Information, insbesondere Codes, insbesondere mittels RFID oder anderer Nahfeldkommunikation.

In einer vorteilhaften Ausgestaltung wird die erste Erfassung durchgeführt und das dadurch Erfasste der ersten Erfassung mit einem Vorbestimmten verglichen und aufgrund des Vergleichs als eine autorisierte oder nicht autorisierte Erfassung gewertet, insbesondere wenn Teile des Erfassten der ersten Erfassung mit dem Vorbestimmten übereinstimmt. Das Vorbestimmte kann durch und/oder auf ein(em) Erfassungsgerät, insbesondere Eingabegerät, oder auf einem anderen Gerät oder vom Erfassungsgerät entfernten Gerät, beispielswiese einem Server oder in einer Datenbank, vorbestimmt sein, wobei bei der ersten Erfassung ein Vergleich des Erfassten der ersten Erfassung mit einem auf einem anderen Gerät oder entfernt Vorbestimmten zum Beispiel unter Verwendung von Kommunikationsmitteln des Erfassungsgeräts bewirkt wird.

In einer weiteren Alternative oder zusätzlich wird aus dem Erfassten eine Information extrahiert und angezeigt, insbesondere auf dem Erfassungsgerät. Insbesondere beinhaltet das Extrahieren ein Entschlüsseln, insbesondere mit einem Schlüssel, der im Erfassungsgerät gespeichert ist oder in dieses eingegeben oder mit diesem erfasst wird.

Das erfindungsgemäße Verfahren beinhaltet zudem und insbesondere daraufhin das Initialisieren eines von der ersten Erfassung abhängigen und/oder durch die erste Erfassung ausgelösten Autorisierungsvorgangs. Vorteilhafterweise ist das Verfahren so ausgebildet, dass der Autorisierungsvorgang durch, und insbesondere nur durch, eine vorherige erste, insbesondere autorisierte, Erfassung initialisiert bzw. ausgelöst wird. Der Autorisierungsvorgang wird insbesondere in Abhängigkeit der ersten Erfassung initialisiert, wobei die Abhängigkeit die kausale Abhängigkeit, also Kausalität, und/oder die inhaltliche Abhängigkeit umfasst, wobei bei einer kausalen Abhängigkeit die Erfassung die Ursache und das Initialisieren des Autorisierungsvorgangs die Wirkung in der Beziehung dieser Kausalität darstellt und wobei bei einer inhaltlichen Abhängigkeit der Autorisierungsvorgang, insbesondere dessen Inhalt und/oder Parameter, zumindest teilweise Teile der ersten Erfassung, insbesondere deren Inhalt und/oder Parameter, umfasst und/oder der der Autorisierungsvorgang, insbesondere dessen Inhalt und/oder Parameter mittels der ersten Erfassung, insbesondere durch den Inhalten und/oder die Parameter der ersten Erfassung, zumindest teilweise angepasst und/oder bestimmt wird. Beispielsweise ist in dem erfindungsgemäßen Verfahren somit vorgesehen, dass nach einer ersten Erfassung, insbesondere einer autorisierten Erfassung, und/oder durch die erste Erfassung, insbesondere einer autorisierten Erfassung, der Autorisierungsvorgang initialisiert wird, insbesondere eine Berechtigungsanfrage generiert wird. So kann das erste Erfasste eine ID sein und kann mittels der ID aus einer Datenbank eine Emailadresse ausgelesen werden und der Autorisierungsvorgang eine E-Mail, insbesondere mit Autorisierungsanfrage, insbesondere Berechtigungsanfrage, als Inhalt, an diese Emailadresse senden. Vorteilhafterweise kann zudem der Autorisierungsvorgang nicht nur in dem Sinne abhängig von der ersten Erfassung sein, dass der Autorisierungsvorgang, insbesondere die Initialisierung dessen, durch die erste Erfassung, insbesondere einer autorisierten Erfassung, ausgelöst wird, sondern auch, in dem Sinne abhängig von der ersten Erfassung sein, dass mittels der Erfassung auch Inhalte und/oder Parameter an den Autorisierungsvorgang übertragen werden. Dies hat zum einen den Vorteil, dass ein Autorisierungsvorgang erst ausgelöst wird, wenn eine erste Erfassung, die eine Berechtigung zum Initialisieren des Autorisierungsvorgangs umfasst, durchgeführt wurde und/oder erfolgte, und zum anderen den Vorteil, dass der Autorisierungsvorgang mittels der ersten Erfassung angepasst werden kann. Der erfindungsgemäße Autorisierungsvorgang weist die Möglichkeit zumindest einer bestätigenden und einer ablehnenden Rückmeldung, insbesondere durch mindestens einen Benutzer und/oder generiert mittels eines, insbesondere berechtigten, Eingriffssystems auf. Der Autorisierungsvorgangs weist somit die Möglichkeit zumindest einer bestätigenden und einer ablehnenden Rückmeldung durch einen Benutzer und/oder generiert mittels eines Eingriffssystems auf. Beispielsweise ermöglicht der erfindungsgemäße Autorisierungsvorgang, dass ein Benutzter die bestätigende und die ablehnende Rückmeldung bewirkt oder zusätzlich oder alternativ dass das Eingriffssystem die bestätigende und die ablehnende Rückmeldung generiert. Vorteilhafterweise weist der Autorisierungsvorgangs die Möglichkeit zumindest einer bestätigenden und einer ablehnenden Rückmeldung durch einen Benutzer und/oder generiert mittels eines Eingriffssystems auf.

Vorteilhafterweise handelt es sich bei dem mindestens einen Benutzer um mindestens einen Berechtigten, der innerhalb des Autorisierungsvorgangs die Freigabe der geschützten Information veranlassen und/oder unterbinden kann. Vorteilhafterweise handelt es sich bei der mittels des, insbesondere berechtigten, Eingriffssystems generierten Rückmeldung um eine Rückmeldung innerhalb des Autorisierungsvorgangs, insbesondere um die bestätigende Rückmeldung, insbesondere mit einer Beschränkung, insbesondere der Freigabe, insbesondere Beschränkung des Inhalts und/oder Umfangs der freigegebenen geschützten Funktion, insbesondere ersten geschützten Funktion. Vorteilhafterweise handelt es sich bei dem, insbesondere berechtigten, Eingriffssystem, das die Rückmeldung generiert, um ein vorbestimmtes Eingriffssystem, welches dazu eingerichtet ist, im Rahmen des Autorisierungsvorgangs ohne Interaktion des mindestens einen Benutzers in den Autorisierungsvorgang einzugreifen. Insbesondere ist das Verfahren so ausgestaltet, dass das Eingriffssystem berechtigt und in der Lage ist in zahlreiche Autorisierungsvorgänge, insbesondere mit unterschiedlichen berechtigten Benutzern und/oder unterschiedlichen Freigaben einzugreifen

Vorteilhafterweise weist der Autorisierungsvorgangs die Möglichkeit der Generierung der Rückmeldung mittels des Eingriffssystems auf, wobei das Eingriffssystem die Generierung insbesondere mittels eines Protokolls, insbesondere eines vorbestimmten Protokolls, bewirkt und/oder in dem Eingriffssystem ein, insbesondere vorbestimmtes, Protokoll implementiert ist, wobei das Protokoll insbesondere in dem die Rückmeldung generierenden Eingriffssystem implementiert ist und/oder eingerichtet ist, einem Nutzer im Rahmen des Autorisierungsvorgangs einen Eingriff in den Autorisierungsvorgang zu erlauben.

Vorteilhafterweise erfolgt der Eingriff im Rahmen des Autorisierungsvorgangs mittels eines, insbesondere vorbestimmten und/oder berechtigten, Nutzers, beispielsweise eines zertifizierten und/oder vorbestimmten Nutzers, beispielsweise eines Organs oder Angestellten einer Krankenkasse oder einer Gesundheitsbehörde.

Vorteilhafterweise erfolgt der Eingriff im Rahmen des Autorisierungsvorgangs und/oder zusätzlich oder alternativ mittels eines Protokolls, insbesondere eines vorbestimmten Protokolls, wobei das Protokoll insbesondere in dem die Rückmeldung generierenden Eingriffssystem implementiert ist und/oder eingerichtet ist, dem Nutzer im Rahmen des Autorisierungsvorgangs einen Eingriff in den Autorisierungsvorgang zu erlauben.

Vorteilhafterweise handelt es sich bei dem Protokoll um ein Sicherheitszugriffsprotokoll und/oder Sicherheitseingriffsprotokoll, insbesondere um eine Break-The-Glass Prozedur, das und/oder die insbesondere eingerichtet ist, im Rahmen des Autorisierungsvorgangs einen Eingriff in Autorisierungsvorgang zuzulassen, insbesondere für den, insbesondere berechtigten, Nutzer, beispielsweise den Administrator des die Rückmeldung generierenden Eingriffssystems und/oder einem Gesundheitsdienstleister und/oder einer Gesundheitsorganisation.

Vorteilhafterweise ist das Eingriffssystem eingerichtet mittels eines Zugangs für den, insbesondere vorbestimmten und/oder berechtigten, Nutzer in den Autorisierungsvorgang einzugreifen, insbesondere diesen zu überschreiben. Insbesondere erfolgt dies mittels des Protokolls. Insbesondere ist das Eingriffssystem dazu eingerichtet innerhalb des Autorisierungsvorgangs eine, insbesondere ausschließlich eine, zumindest teilweise bestätigende Rückmeldung zu generieren. Vorteilhafterweise ist das Eingriffssystem mit dem Protokoll, welches insbesondere eingerichtet ist, in den Autorisierungsvorgang einzugreifen, dazu eingerichtet, dass es, insbesondere dem, insbesondere vorbestimmten und/oder berechtigten, Nutzer, ermöglicht, innerhalb des Autorisierungsvorgangs eine bestätigende Rückmeldung zu generieren, insbesondere ohne dass der Benutzer eine bestätigende, insbesondere bedingte bestätigende, Rückmeldung erwirkt und/oder die genierte beschränkte bestätigende Rückmeldung bestätigt. Dies kann, wie auch dem Stand der Technik bekannt, in Notsituationen hilfreich sein.

Vorteilhafterweise ist das erfindungsgemäße Verfahren und/oder System eingerichtet, dass im Rahmen des Autorisierungsvorgangs die Möglichkeit eines Eingriffs in das Autorisierungsverfahren durch, insbesondere vorbestimmte und/oder berechtigte, Nutzer vorgesehen ist, insbesondere mittels des vorbestimmten Protokolls. Bei dem Protokoll handelt es sich insbesondere um die Break-The-Glass Prozedur. Insbesondere bewirkt ein Eingriff durch den, insbesondere vorbestimmten und/oder berechtigten, Nutzer eine bestätigende Freigabe einer geschützten, insbesondere der ersten und/oder einer beschränkt geschützten, Funktion. Insbesondere umfasst der Eingriff im Rahmen des Autorisierungsvorgangs ein Überschreiben und/oder Umgehen der vorbestimmten Bewirkung einer Rückmeldung durch den Benutzer. Beispielsweise kann ein solcher Eingriff eine wie im Stand der Technik bekannte Break-The-Glass Prozedur sein.

Vorteilhafterweise zeichnet sich der mindestens eine berechtigte Benutzer und/oder berechtigte Nutzer und/oder das berechtigte Eingriffssystem durch das Merkmal aus, dass dieser und/oder dieses vorbestimmt und/oder vorgesehen ist eine geschützte Freigabe mittels des erfindungsgemäßen Verfahren und/oder System im Rahmen des Autorisierungsvorgangs zu bewirken und/oder zu veranlassen. Hierzu bedarf es beispielsweise, dass der mindestens eine berechtigte Benutzter als Berechtigter in dem erfindungsgemäßen Verfahren als solcher erfasst und/oder vorbestimmt ist und/oder eines Berechtigungsmittel zur Freigabe einer geschützten Funktion im Rahmen des Autorisierungsvorgangs. Beispielsweise handelt es sich bei dem Berechtigungsmittel um ein Kennwort, insbesondere ein vorbestimmtes Kennwort, und/oder ein eindeutiges, vorbestimmtes und/oder dem Benutzter zugeordnetes Kennzeichen. Insbesondere sieht das erfindungsgemäße Verfahren den Schritt der Abfrage und/oder Prüfung eines Autorisierungsmittels und/oder Berechtigungsmittel des Benutzers und/oder des Eingriffssystems und/oder des Nutzers vor und sieht insbesondere bei einer erfolgreichen und/oder positiven Überprüfung den Benutzer, das Eingriffssystem und/oder Nutzer als berechtigt an. Insbesondere ist das erfindungsgemäße Verfahren so eingerichtet, dass es Rückmeldungen von nicht Berechtigten nicht berücksichtigt und/oder nicht Berechtigten nicht die Möglichkeit einer Rückmeldung bietet. Beispielsweise ist dem berechtigten Eingriffssystem ein im Rahmen des Autorisierungsvorgangs wirkendes Zugriffsrecht auf das erfindungsgemäße Verfahren und/oder System eingeräumt und/oder weist das erfindungsgemäße Verfahren und/oder System ein im Rahmen des Autorisierungsvorgangs wirkendes Zugriffsrecht für das berechtigte Eingriffssystem auf.

Vorteilhafterweise ist ein berechtigter Nutzer ein Berechtigter und eine zertifizierte Person und/oder Institution, die befugt ist, auf das berechtigte Eingriffssystem zuzugreifen und im Rahmen des Autorisierungsvorgangs mittels des Eingriffssystems auf das erfindungsgemäße Verfahren und/oder System einzuwirken.

Vorteilhafterweise ist die bestätigende und/oder ablehnende Rückmeldung, insbesondere deren, insbesondere vorgegebener, Inhalt und/oder Parameter abhängig von der ersten Erfassung, insbesondere deren Inhalt und/oder Parameter. Insbesondere wird ein Teil des Inhalts und/oder der Parameter der Rückmeldung durch die erste Erfassung, insbesondere deren Inhalt und/oder Parameter, vorgegeben.

Beispielsweise kann ein erfindungsgemäßes Initialisieren eines Autorisierungsvorgangs eine Generierung einer Berechtigungsanfrage sein. Beispielsweise ist der Autorisierungsvorgang eine Anfrage, ob eine Funktion oder eine Information freigegeben werden soll, wobei darauf bestätigend, also im Sinne von "JA", oder ablehnend, also im Sinne von "NEIN", geantwortet werden kann.

Vorteilhafterweise wird der Autorisierungsvorgang, insbesondere die Berechtigungsanfrage, versendet und/oder, insbesondere an einem Anzeigegerät, insbesondere zur Freigabe durch einen Benutzer und/oder Inhaber der geschützten Funktion/Information, angezeigt.

Bei einem Ausbleiben sowohl einer ablehnenden als auch einer bestätigenden Rückmeldung und insbesondere auch einer mittels des Eingriffssystems generierten Rückmeldung im Rahmen des Autorisierungsvorgangs, insbesondere innerhalb einer, insbesondere vorbestimmten, Zeitspanne, während des erfindungsgemäßen Verfahrens wird die Freigabe mindestens einer ersten Funktion, insbesondere Ausgabe mindestens einer ersten Information, bewirkt. Vorteilhafterweise wird zusätzlich bei einem Ausbleiben jeglicher Rückmeldung und/oder eines, insbesondere berechtigten und/oder unberechtigten, Eingriffs, insbesondere in den Autorisierungsvorgang, und/oder eines Außerkraftsetzens, Umgehens und/oder Überschreibens des Autorisierungsvorgangs im Rahmen des Autorisierungsvorgangs, insbesondere innerhalb einer, insbesondere vorbestimmten, Zeitspanne, während des erfindungsgemäßen Verfahrens die Freigabe mindestens einer ersten Funktion, insbesondere Ausgabe mindestens einer ersten Information, bewirkt. Bisher war bei einem Ausbleiben irgendeiner Rückmeldung im Stand der Technik vorgesehen, entweder keinerlei weitere Vorgänge oder keine Freigabe zu bewirken. Durchgeführt wurden in der Regel nur Rückmeldungen, dass eine Autorisierungsanfrage nicht bestätigend und/oder mit einem vorgesehenen Code beantwortet wurde. Dies hat zur Folge, wie die Erfinder erkannt haben, dass benötigte Freigaben bzw. Informationen nicht freigegeben wurden, was in bestimmten Situationen nachteilig sein kann, beispielsweise wenn Ersthelfer oder Rettungspersonal Gesundheitsdaten anfragen und diese nicht erhalten, weil die zur Autorisierung berechtigte und/oder fähige Person nicht ansprechbar und/oder nicht handlungsfähig ist, was insbesondere bei Notfällen diese Person betreffend und entsprechender Anfrage von Gesundheitsdaten dieser Person gegeben ist.

Für derartige Notfälle ist aus dem Stand der Technik die Möglichkeit des Eingriffs in den Autorisierungsvorgangs bekannt, insbesondere unter dem Begriff "Break-The-Glass". Dies ist vorteilhafterweise aber als bestätigende Rückmeldung, wenn auch als eine beschränkt bestätigende Rückmeldung, insbesondere mit daraus resultierender eingeschränkter Freigabe, zu werten. Die Einführung einer solchen Option beschränkt aber die Entscheidungsfreiheit des Benutzers und/oder die Flexibilität des Systems und/oder Verfahrens. Dieses Problem löst die Erfindung, indem es bei einer ablehnenden Antwort vorteilhafterweise auch eine solche Möglichkeit des Eingriffs oder der Freigabe verhindert und/oder sperrt und insbesondere eine Break-The-Glass Möglichkeit außer Kraft setzt. So kann der Benutzer die Freigabe wirksam verhindern, und kann gleichzeitig in Notfällen die Freigabe einer ersten Funktion und/oder Ausgabe der ersten Information ermöglicht werden.

Vorteilhafterweise führt nach einer ablehnenden Rückmeldung, insbesondere durch den mindestens einen Benutzer, im Rahmen des Autorisierungsvorgangs eine nachfolgende bestätigende Rückmeldung, insbesondere eine auf die ablehnende Rückmeldung folgende bestätigende Rückmeldung, im Rahmen des Autorisierungsvorgangs nicht zum Bewirken der Freigabe der mindestens einen ersten und/oder zweiten Funktion im Rahmen des Autorisierungsvorgangs. Vorteilhafterweise führt nach einer ablehnenden Rückmeldung, insbesondere durch den mindestens einen Benutzer, im Rahmen des Autorisierungsvorgangs eine im Rahmen des Autorisierungsvorgangs nachfolgende und mittels des Eingriffssystems generierte, insbesondere auf die ablehnende Rückmeldung folgende, Rückmeldung im Rahmen des Autorisierungsvorgangs nicht zum Bewirken der Freigabe der mindestens einen ersten und/oder zweiten Funktion.

Vorteilhafterweise werden nach einer ablehnenden Rückmeldung, insbesondere durch den mindestens einen Benutzer, im Rahmen des Autorisierungsvorgang bestätigende Rückmeldungen im Rahmen des Autorisierungsvorgang ignoriert. Mit Vorteil bewirkt eine ablehnende Rückmeldung des Eingriffssystem nicht zu einer solchen Missachtung späterer bestätigender Rückmeldung. Bevorzugt ist das Verfahren aber so eingerichtet, dass durch das Eingriffssystem nur bestätigende Rückmeldungen vorgesehen sind.

Vorteilhafterweise erfolgt die Rückmeldung im Rahmen des Autorisierungsvorgangs durch einen Benutzer und/oder erfolgt im Rahmen des Autorisierungsvorgangs eine mittels des Eingriffssystems, insbesondere automatisch, generierte Rückmeldung. Insbesondere wird die Rückmeldung mittels des Eingriffssystems automatisch generiert, wobei die automatische Generierung die Generierung ohne Interaktion eines Benutzers umfasst, wobei insbesondere lediglich, insbesondere vorbestimmte und/oder berechtigte, Nutzer die automatische Generierung auslösen. Insbesondere erfolgt die Generierung der Rückmeldung durch das Eingriffssystem derart automatisch, dass allein durch eine Interaktion des, insbesondere berechtigten, Nutzers mit dem Eingriffssystem das Eingriffssystem eine Rückmeldung im Rahmen des Autorisierungsverfahrens bewirkt.

Vorteilhafterweise ist das erfindungsgemäße Verfahren und/oder System mit der Möglichkeit eingerichtet, dass im Rahmen des Autorisierungsvorgangs, insbesondere durch eine bestätigende Rückmeldung, ein Überschreiben des Autorisierungsvorgangs, ein Umgehen des Autorisierungsvorgangs und/oder eine Außerkraftsetzung des Autorisierungsvorgangs im Rahmen des Autorisierungsvorgangs aufgrund des vorbestimmten Protokolls, insbesondere einer Break-The-Glass Prozedur, und/oder mittels des, insbesondere vorbestimmten und/oder berechtigten, Nutzers erfolgt.

Vorteilhafterweise ist ein Eingriff ein Eingriff in den Autorisierungsvorgang im Rahmen des Autorisierungsvorgang. Vorteilhafterweise erfolgt der Eingriff aufgrund und/oder mittels des vorbestimmten Protokolls. Vorteilhafterweise ist der Eingriff ein Überschreiben und/oder eine Umgehung des Autorisierungsvorgangs, insbesondere einer Logik und/oder Berechtigungslogik und/oder eins Berechtigungsprotokolls des Autorisierungsvorgangs, insbesondere ein Überschreiben und/oder Umgehen durch einen, insbesondere vorbestimmten, Nutzer, insbesondere mittels des vorbestimmten Eingriffssystems, das die Rückmeldung generiert. Insbesondere ist der Eingriff eingerichtet, zumindest eine bestätigende Rückmeldung im Rahmen des Autorisierungsvorgangs zu bewirken oder erfolgt er im Rahmen einer solchen. Insbesondere umfasst die Logik und/oder Berechtigungslogik des Autorisierungsvorgangs die Bedingungen aufgrund welcher Bestätigung welches Benutzers eine, insbesondere eine vorbestimmte, bestätigende Rückmeldung bewirkt wird. Insbesondere umfasst das vorbestimmte Protokoll und/oder das vorbestimmte Protokoll eine Break-the-Glass Prozedur.

Vorteilhafterweise ist das Außerkraftsetzen, Umgehen und/oder Überschreiben des Autorisierungsvorgangs mit der Möglichkeit eingerichtet, dass durch das Außerkraftsetzen, Umgehen und/oder Überschreiben des Autorisierungsvorgangs zumindest eine bestätigende Rückmeldung im Rahmen des Autorisierungsvorgangs bewirkt wird.

Vorteilhafterweise handelt es sich bei der mittels des Eingriffssystems generierten bestätigenden Rückmeldung um eine Rückmeldung mit einer Beschränkung, insbesondere einer Beschränkung der Freigabe einer geschützten Funktion, so dass insbesondere geschützte Funktionen lediglich im beschränkten Umfang freigegeben werden. Diese geschützten Funktionen können mehr oder weniger geschützte Funktionen als die erste und/oder zweite geschützte Funktion umfassen. Vorteilhafterweise umfasst die aufgrund der mittels des Eingriffssystems generierten bestätigenden Rückmeldung bewirkten Freigabe der geschützten Funktion zumindest die Freigabe der ersten geschützten Funktion, insbesondere jedoch nicht die Freigabe aller geschützten Funktionen der zweiten geschützten Funktion.

Erfindungsgemäß beinhaltet die mindestens eine erste Funktion eine geschützte Funktion, insbesondere eine der mindestens einen geschützten Funktion, insbesondere die erste geschützte Funktion, insbesondere beinhaltet die Ausgabe mindestens einer ersten Information die Ausgabe einer geschützten Information, insbesondere eine der mindestens einen geschützten Information, insbesondere der ersten geschützten Information.

Alternativ oder zusätzlich ist erfindungsgemäß die mindestens eine erste Funktion, insbesondere deren Umfang und/oder Inhalt und/oder Parameter, unabhängig vom Autorisierungsvorgang, insbesondere beinhaltet die mindestens eine erste Information vom Autorisierungsvorgang unabhängige Information(en). Insbesondere ist die mindestens eine erste Funktion, insbesondere die mindestens eine erste Information, insbesondere deren Umfang und/oder Inhalt und/oder Parameter, unabhängig von den Daten der Berechtigungsanfrage und/oder von der Berechtigungsanfrage und/oder nicht in den Daten der Berechtigungsanfrage enthalten.

Der Vorteil der ersten Alternative, also des Beinhaltens einer geschützten Funktion/Information in der ersten Funktion/Information, besteht darin, dass trotz eines Fehlens einer Bestätigung, das Ausbleiben einer Rückmeldung, solange eben auch keine ablehnende Rückmeldung vorhanden ist, nicht als eine Ablehnung interpretiert wird, sondern geschützte Funktionen freigeben werden, insbesondere geschützte Informationen ausgegeben werden. Dies könnte von Vorteil sein, wenn eine bestätigende Rückmeldung wegen anderer Umstände nicht erfolgen kann, aber gewollt ist oder sein mag. Es kann zum Beispiel sein, dass eine bestätigende Rückmeldung wegen technischer Störungen nicht möglich ist, oder das Medium, das eine Bestätigung vornehmen kann, verhindert und/oder fehlerhaft ist oder die zur Bestätigung fähige und/oder berechtigte Person ver- und/oder gehindert ist.

Der Vorteil der zweiten Alternative, also der Unabhängigkeit der ersten Funktion/Information von dem Autorisierungsvorgang, ist, dass die erste Funktion eine Funktion ist, die nicht abhängig, insbesondere nicht kausal und/oder inhaltlich abhängig, ist von dem Autorisierungsvorgang, wodurch die erste Funktion demnach zumindest eine weitere Funktion enthält, die nicht von dem Autorisierungsvorgang abhängig ist, somit beispielsweise die erste Funktion nicht lediglich die Ausgabe der Information, dass eine Autorisierungsanfrage gesendet wurde, umfasst, sondern beispielsweise eine geschützte oder andere Funktion aufweist.

Vorteilhafterweise wird bei Ausbleiben sowohl einer bestätigenden als auch ablehnenden Rückmeldung zusätzlich, neben der Freigabe der mindestens einen ersten Funktion, eine Freigabe einer ungeschützten Funktion, insbesondere Ausgabe einer ungeschützten Information, bewirkt.

Beispielsweise kann die mindestens eine erste Funktion derart unabhängig vom Autorisierungsvorgang sein, dass diese mindestens eine erste Funktion eine vom Autorisierungsvorgang unabhängige Funktion umfasst, insbesondere der Umfang und/oder Inhalt und/oder Parameter und/oder die Eigenschaft der mindestens einen ersten Funktion eine Funktion mit Umfang und/oder Inhalt(en) und/oder Parameter(n) und/oder Eigenschaft(en) unabhängig des Autorisierungsvorgangs umfasst. Beispielsweise beinhaltet die mindestens eine erste Information eine vom Autorisierungsvorgang unabhängige Information derart, dass der Inhalt und/oder Umfang der mindestens einen ersten Information zumindest eine Information enthält, welche von dem Inhalt und/oder Umfang, und/oder Parameter des Autorisierungsvorgangs unabhängig ist.

Mittels des erfindungsgemäßen Verfahrens wird zum Beispiel erzielt, dass trotz fehlender Ablehnung und Zustimmung eine Freigabe einer Funktion, insbesondere Ausgabe einer Information erfolgt, insbesondere einer geschützten Funktion und/oder geschützten Information, erfolgt und/oder bewirkt wird. Dies kann zum Beispiel dann gewollt sein, wenn das System, beispielsweise das Eingriffssystem, oder eine Person, beispielsweise der Benutzter, die eine Freigabe oder Ausgabe durch Ablehnung oder Bestätigung autorisieren vermag, gehindert oder verhindert ist, aber im Grund die Freigabe oder Ausgabe den Umständen entsprechend gewollt oder zumindest hinnehmbar ist.

Insbesondere wird in dem erfindungsgemäßen Verfahren aufgrund der ersten Erfassung und/oder der Initialisierung des Autorisierungsvorgangs eine gesteuerte und/oder gesicherte und/oder kontrollierte Freigabe mindestens einer geschützten Funktion, insbesondere Ausgabe mindestens einer geschützten Information, bewirkt. Insbesondere wird die Steuerung und/oder Sicherung und/oder Kontrolle der Freigabe durch die erste Erfassung, insbesondere dem Vergleich der ersten Erfassung mit dem Vorbestimmten, und/oder den Autorisierungsvorgang, insbesondere der Berechtigungsanfrage, und/oder Rückmeldung, insbesondere bestätigender Rückmeldung, bewirkt. Durch diese, insbesondere Mehrzahl an, Sicherungsstufe(n) wird eine Sicherung der geschützten Funktionen, insbesondere Informationen, und zugleich zweckmäßigen Freigabe derer gewährleistet, wobei das Verfahren durch weitere Autorisierungsvorgänge ergänzt werden kann. Beispielsweise kann mittels der Erfassung einer speziellen vorbestimmten Information (beispielsweise ein Passwort oder Schlüssel zur Entschlüsselung) bei Ausbleiben einer Rückmeldung im Rahmen des Autorisierungsvorgangs eine geschützte Funktion freigeben, insbesondere eine geschützte Information ausgegeben, werden.

Insbesondere ist es bei dem erfindungsgemäßen Verfahren unwesentlich, inwiefern die einzelnen Verfahrensschritte physikalisch, physisch und/oder örtlich an einem gemeinsamen Ort und/oder auf einem gemeinsamen Gerät durchgeführt werden. Insbesondere kann die Freigabe, die Hinterlegung der Funktion, Ausgabe der Information, erste Erfassung, das Initialisieren des Autorisierungsvorgangs, Ablehnen oder Bestätigung des Autorisierungsvorgangs, Generierung einer Rückmeldung, Empfangen einer Rückmeldung und/oder die Ausführung der Funktion örtlich, physisch und/oder physikalisch jeweils zueinander getrennt sein.

Vorteilhafterweise ist die geschützte Funktion, insbesondere die geschützte Information, extern, beispielsweise auf einem entfernten Server, oder auf einem, insbesondere die erste Erfassung durchführenden, Ausführungssystem, insbesondere einem nachfolgend näher erläutertem erfindungsgemäßen System, hinterlegt und/oder wird die geschützte Funktion, insbesondere die geschützte Information, extern, beispielsweise auf einem entfernten Server, oder auf einem, insbesondere die erste Erfassung durchführenden, Ausführungssystem, insbesondere einem nachfolgend näher erläutertem erfindungsgemäßen System ausgeführt. In einer alternativen Ausführungsform oder zusätzlich kann die geschützte Funktion oder Parameter für deren Ausführung, insbesondere die geschützte Information, zumindest teilweise in dem Erfassten beinhaltet sein und abhängig von der Rückmeldung des Autorisierungsvorgangs verfügbar gemacht werden. So kann die erste und/oder zweite Funktion und/oder Information teilweise oder ganz, insbesondere verschlüsselt im Erfassten enthalten sein. So kann sie, beispielsweise an einen Server, im oder vor dem Autorisierungsvorgang übermittelt und abhängig von der Rückmeldung der entsprechende Teil bereitgestellt und/oder entschlüsselt werden.

Insbesondere wird bei einer extern oder einer auf einem, insbesondere die erste Erfassung durchführenden, Ausführungssystem hinterlegten, geschützten Funktion, insbesondere Information, aufgrund einer bestätigenden Rückmeldung oder einem Ausbleiben einer Rückmeldung aufgrund vorbestimmter Bestimmungen und/oder Richtlinien, die beispielsweise durch die Erfassung bestimmt oder extern vorbestimmt werden, zumindest ein Teil der extern hinterlegten geschützten Funktion, insbesondere die Ausgabe zumindest eines Teils der extern hinterlegten Information, freigegeben. Insbesondere wird die Freigabe der extern oder auf dem Ausführungssystem hinterlegten, geschützten Funktion aufgrund von vorbestimmten Bestimmungen und/oder Richtlinien extern oder durch das Ausführungssystem freigegeben.

Insbesondere wird bei einer durch und/oder mittels die/der Erfassung hinterlegten, insbesondere geschützten, Funktion, insbesondere Information, insbesondere aufgrund einer bestätigenden Rückmeldung oder einem Ausbleiben einer Rückmeldung, insbesondere aufgrund vorbestimmter Bestimmungen und/oder Richtlinien, die extern vorbestimmt sind, insbesondere durch Bestimmungen und/oder Richtlinien, die durch die Autorisierungsanfrage von einem entfernten System, insbesondere Server oder erfindungsgemäßen System, übermittelt und/oder bestimmt werden, zumindest ein Teil der durch und/oder mittels die/der Erfassung hinterlegten, insbesondere geschützten, Funktion, insbesondere die Ausgabe zumindest eines Teils der durch und/oder mittels die/der Erfassung hinterlegten Information, freigegeben.

Vorteilhafterweise ist die Freigabe der mindestens einen ersten Funktion, insbesondere Ausgabe der mindestens einen ersten Information, unabhängig von Eigenschaften und/oder Parametern und/oder Daten und/oder Inhalt und/oder Umfang des Autorisierungsvorgangs und/oder unabhängig von Daten der Berechtigungsanfrage und/oder der Berechtigungsanfrage.

Vorteilhafterweise beinhaltet die Freigabe der mindestens einen ersten Funktion, insbesondere Ausgabe der mindestens einen ersten Information, lediglich einen ersten Teil der geschützten Funktion, insbesondere Ausgabe lediglich eines ersten Teils einer geschützten Information.

Insbesondere umfasst zumindest ein erster Anteil der in der mindestens einen ersten Funktion beinhaltende erste Teil der geschützten Funktion eine vorbestimmte und/oder vorausgewählte und/oder von dem Autorisierungsvorgang, insbesondere dessen Eigenschaften und/oder Parametern und/oder Daten und/oder Inhalt und/oder Umfang, abhängige und/oder daraus ausgewählte geschützte Funktion. Bei einer solchen Gestaltung kann die mindestens eine erste Funktion zu einem Teil geschützten Funktionen, welche auch eine vorbestimmte und/oder vorausgewählte und/oder von dem Autorisierungsvorgang abhängige geschützte Funktion beinhaltet, sowie zu einem weiteren Teil eine von dem Autorisierungsvorgang unabhängige Funktion beinhalten.

Insbesondere umfasst zumindest ein erster Anteil der in der mindestens einen ersten Information beinhaltende erste Teil der geschützten Information eine vorbestimmte und/oder vorausgewählte und/oder von dem Autorisierungsvorgang, insbesondere dessen Eigenschaften und/oder Parametern und/oder Daten und/oder Inhalt und/oder Umfang, abhängige und/oder daraus ausgewählte geschützte Information. Bei einer solchen Gestaltung kann die mindestens eine erste Information zu einem Teil geschützte Information, welche auch eine vorbestimmte und/oder vorausgewählte und/oder von dem Autorisierungsvorgang abhängige geschützte Information, sowie zu einem weiteren Teil eine von dem Autorisierungsvorgang unabhängige Information beinhalten.

Durch eine solche Ausgestaltung bzw. Limitierung der ersten Funktion/Information wird gewährleistet, dass ein Teil der geschützten Funktion/Information freigegeben/ausgegeben wird, sodass zumindest ein Teil dieser geschützten Funktionen/Information zugänglich ist, der Rest der geschützten Funktion allerdings weiterhin gesichert ist.

Vorteilhafterweise wird bei bestätigender Rückmeldung im Rahmen des Autorisierungsvorgangs, insbesondere der Berechtigungsanfrage, die Freigabe mindestens einer zweiten Funktion, insbesondere Ausgabe mindestens einer zweiten Information, bewirkt. Zumindest ein Teil der mindestens einen zweiten Funktion, insbesondere Ausgabe der mindestens einen zweiten Information, ist eine geschützte Funktion, insbesondere geschützte Information, vorzugsweise eine der mindestens einen geschützten Funktionen, insbesondere eine der mindestens einen geschützten Informationen, insbesondere ein zweiter Teil der geschützten Funktion, insbesondere ein zweiter Teil der geschützten Information. Die geschützte Funktion der zweiten Freigabe, insbesondere die zweite geschützte Information, vorzugsweise der zweite Teil der geschützten Funktion der mindestens einen zweiten Funktion, insbesondere zweite Teil der geschützten Information der mindestens einen zweiten Information, ist nicht identisch mit der geschützten Funktion der ersten Freigabe, insbesondere ersten geschützten Information, dem ersten Teil der geschützten Funktion der mindestens einen ersten Funktion, insbesondere ersten Teil der geschützten Information der mindestens einen ersten Information. Bezüglich des nicht identisch kommt es lediglich darauf an, dass die Menge bzw. der Inhalt der geschützten Funktion der zweiten Freigabe, insbesondere des zweiten Teils der geschützten Funktionen, insbesondere Informationen, der zweiten Funktion, insbesondere Information, nicht zu 100% der geschützten Funktion der ersten Freigabe, insbesondere des ersten Teils der geschützten Funktionen, insbesondere Informationen, der ersten Funktion, insbesondere Information, entspricht, wobei insbesondere die beiden Mengen bzw. beide Teile jeweils überlappen bzw. sich jeweils überschneiden können.

Vorteilhafterweise ist zumindest ein zweiter Anteil des zweiten Teils der geschützten Funktion der mindestens einen zweiten Funktion eine von der Rückmeldung im Rahmen des Autorisierungsvorgangs und/oder eine von und/oder auf Parametern und/oder Inhalt der Rückmeldung im Rahmen des Autorisierungsvorgangs abhängige und/oder basierende geschützte Funktion. Vorteilhafterweise beinhaltet zumindest ein zweiter Anteil des zweiten Teils der geschützten Information der mindestens einen zweiten Information eine von der Rückmeldung im Rahmen des Autorisierungsvorgangs und/oder eine von und/oder auf Parametern und/oder Inhalt der Rückmeldung im Rahmen des Autorisierungsvorgangs abhängige und/oder basierende geschützte Information. Insbesondere schließt eine solche vorteilhafte Weiterbildung nicht aus, dass neben der von der Rückmeldung und/oder eine von und/oder auf Parametern und/oder Inhalt der Rückmeldung abhängigen und/oder basierenden, geschützten Funktion bzw. Information auch andere geschützte Funktionen bzw. Informationen und andere nicht-geschützte Funktionen bzw. Informationen Teil der mindestens einen zweiten Funktion bzw. Information ist.

Vorteilhafterweise wird bei einer ablehnenden Rückmeldung im Rahmen des Autorisierungsvorgangs, insbesondere der Berechtigungsanfrage, keine Freigabe oder eine Freigabe mit einer, insbesondere lediglich einer, dritten Funktion, insbesondere Ausgabe einer, insbesondere lediglich einer, dritten Information, bewirkt.

Vorteilhafterweise ist die dritte Funktion keine geschützte Funktion, insbesondere beinhaltet die dritte Information keine geschützte Information.

Alternativ ist die dritte Funktion eine, insbesondere lediglich eine, von und/oder auf Parametern und/oder Inhalt der Rückmeldung im Rahmen des Autorisierungsvorgangs abhängige und/oder basierende geschützte Funktion, insbesondere dritter Teil der geschützten Funktion, insbesondere beinhaltet die dritte Information einen, insbesondere lediglich eine von und/oder auf Parametern und/oder Inhalt der Rückmeldung im Rahmen des Autorisierungsvorgangs abhängige und/oder basierende geschützte Information, insbesondere dritten Teil der geschützten Information, wobei die geschützte Funktion der dritten Funktion, insbesondere der dritte Teil der geschützten Funktion der dritten Funktion, insbesondere die geschützte Information der dritten Information, insbesondere der dritte Teil der geschützten Information der dritten Information, nicht identisch ist mit der geschützten Funktion der ersten und/oder zweiten Freigabe, insbesondere dem ersten und/oder zweiten Teil der geschützten Funktion der mindestens einen ersten und/oder zweiten Funktion, insbesondere mit dem ersten und/oder zweiten Teil der geschützten Information der mindestens einen ersten und/oder zweiten Information. Bezüglich des nicht identisch kommt es lediglich darauf an, dass die Menge bzw. der Inhalt des dritten Teils der geschützten Funktionen der dritten Funktion, insbesondere dritten Teils der geschützten Informationen der dritten Information, nicht zu 100% der Menge bzw. Inhalt des ersten und/oder zweiten Teils der geschützten Funktionen der ersten und/oder zweiten Funktion, insbesondere des ersten und/oder zweiten Teils der geschützten Informationen der ersten und/oder zweiten Information, entspricht, wobei insbesondere die Mengen bzw. Inhalte jeweils überlappen und/oder sich jeweils überscheiden können.

Vorteilhafterweise wird das erfindungsgemäße Verfahren durch ein Berechtigungsmittel gesteuert und/oder kontrolliert. Insbesondere ist das Berechtigungsmittel Teil eines externen Servers, insbesondere des Servers, der die geschützten Funktionen und/oder Informationen beinhaltet und/oder sichert, oder Teil des Erfassungsgerät.

Vorteilhafterweise umfasst das erfindungsgemäße Verfahren den Schritt eines Bewirkens einer Initialfreigabe, insbesondere Initialausgabe, insbesondere in und/oder auf und/oder durch ein/einem ersten Ausgabegerät und/oder Anzeigegerät oder dem Erfassungsgerät, wobei dieser Schritt insbesondere vor dem Initialisieren des Autorisierungsvorgangs, insbesondere dem Generieren der Berechtigungsanfrage, erfolgt.

Vorteilhafterweise umfasst die Initialfreigabe mindestens eine Initialfunktion, insbesondere Initialausgabe mindestens einer Initialinformation, welche insbesondere unabhängig vom Autorisierungsvorgang ist, insbesondere unabhängig von den Daten der Berechtigungsanfrage und/oder von der Berechtigungsanfrage ist und/oder welche nicht in den Daten der Berechtigungsanfrage enthalten sind.

Insbesondere erfolgt die Freigabe der mindestens einen ersten und/oder zweiten und/oder dritten Funktion und die Initialfreigabe der Initialfunktion auf dem selben Gerät, insbesondere dem ersten Ausgabegerät oder Anzeigegerät oder dem Erfassungsgerät. Insbesondere erfolgt die Ausgabe der mindestens einen ersten und/oder zweiten und/oder dritten Information und die Initialausgabe der Initialinformation auf dem selben Gerät, insbesondere dem ersten Ausgabegerät oder Anzeigegerät oder dem Erfassungsgerät.

Vorteilhafterweise umfasst die Initialfunktion lediglich einen vierten Teil der geschützten Funktion, insbesondere beinhaltet die Initialinformation lediglich einen vierten Teil der geschützten Information, wobei insbesondere dieser vierte Teil der geschützten Funktion der Initialfunktion nicht identisch mit dem ersten und/oder zweiten Teil der geschützten Funktion der ersten und/oder zweiten Funktion ist, insbesondere dieser vierte Teil der geschützten Information der Initialinformation nicht identisch mit dem ersten und/oder zweiten Teil der geschützten Information der ersten und/oder zweiten Information ist.

Vorteilhafterweise wird der Autorisierungsvorgang, insbesondere die Berechtigungsanfrage, durch und/oder abhängig von der/die ersten Erfassung und/oder der/die Initialausgabe und/oder der/die Initialfreigabe und/oder die/den vorbestimmten und/oder gespeicherten Daten und/oder Konfiguration initialisiert, insbesondere generiert.

Vorteilhafterweise wird die Initialisierung des Autorisierungsvorgangs, insbesondere Generierung der Berechtigungsanfrage, zur Benutzerinteraktion und/oder an ein und/oder hin zu einem tragbare(s/n) und/oder mobile(s/n) und/oder mitführ-bare(s/n) und/oder persönliche(s/n) Gerät, insbesondere Anzeigegerät, bewirkt.

Vorteilhafterweise ist das Ausbleiben einer Rückmeldung ein Ausbleiben einer Rückmeldung innerhalb eines vorbestimmten Zeitintervalls, wobei insbesondere der Zeitpunkt der Initialisierung des Autorisierungsvorgangs, insbesondere Generierung der Berechtigungsanfrage, der Anfangspunkt dieses Zeitintervalls ist. Insbesondere stellt das Ausbleiben einer Rückmeldung eine Zeitüberschreitung, insbesondere eine Zeitüberschreitung des vorbestimmten Zeitintervalls dar.

Vorteilhafterweise beinhaltet bei einer, insbesondere bestätigenden, Rückmeldung im Rahmen des Autorisierungsvorgangs, insbesondere der Berechtigungsanfrage, die Rückmeldung mindestens einen, insbesondere mindestens zwei, Parameter, wobei mindestens die erste und/oder zweite und/oder dritte Funktion, insbesondere Information, und/oder zumindest ein Teil davon aufgrund des Parameters ausgewählt wird.

Vorteilhafterweise bewirkt und/oder umfasst die Freigabe der mindestens einen ersten Funktion, insbesondere Ausgabe der mindestens einen ersten Information, und/oder der mindestens einen zweiten Funktion, insbesondere Ausgabe der mindestens einen zweiten Information, und/oder der mindestens einen dritten Funktion, insbesondere Ausgabe der mindestens einen dritten Information, und/oder der mindestens einen vierten Funktion, insbesondere Ausgabe der mindestens einen vierten Information, und/oder die Initialfreigabe der Initialfunktion, insbesondere Initialausgabe der mindestens einen Initialinformation, eine Anzeige und/oder Ausführung der ersten, zweiten, dritten und/oder vierten Funktion und/oder Information und/oder Initialfunktion und/oder Initialinformation, insbesondere auf dem ersten Erfassungsgerät und/oder Ausgabegerät und/oder Anzeigegerät.

Vorteilhafterweise ist das erste Erfassungsgerät und/oder Ausgabegerät und/oder Anzeigegerät ein tragbares und/oder mobiles und/oder mitführbares und/oder persönliches Gerät.

Vorteilhafterweise wird das Initialisieren des Autorisierungsvorgangs, insbesondere Generieren der Berechtigungsanfrage, auf dem Erfassungsgerät oder durch den Gebrauch von Kommunikationsmittel an einem vom Erfassungsgerät und/oder Ausgabegerät entfernten Gerät und/oder an einem Server ausgeführt.

Gelöst wird die Aufgabe auch durch ein System zur, insbesondere gesicherten und/oder gesteuerten und/oder kontrollierten, Freigabe mindestens einer geschützten Funktion, insbesondere ausgebend mindestens eine geschützte Information, wobei das System ein Erfassungsgerät, insbesondere Eingabegerät, zum Erfassen einer ersten Anfrage, ein Kommunikationsmittel zum Senden eines Autorisierungsvorgangs zum zumindest Bestätigen oder Ablehnen der ersten Anfrage, ein Empfangsmittel zum Empfangen einer Rückmeldung über den Autorisierungsvorgang, insbesondere dessen Bestätigung oder Ablehnung, eine Vorrichtung ausweisend und/oder eingerichtet zum Ausführen geschützter Funktionen, insbesondere ein Ausgabegerät eingerichtet zur Anzeige geschützter Information, und ein Freigabemittel zur Freigabe der geschützten Funktionen auf und/oder mittels der Vorrichtung, insbesondere Ausgabe der geschützten Information auf und/der mittels dem/des Ausgabegerät(s), aufweist.

In dem erfindungsgemäßen System ist das Freigabemittel eingerichtet, bei und nach dem Erfassen der ersten Anfrage und Ausbleiben eines Empfangs einer, insbesondere sowohl bestätigenden als auch ablehnenden, Rückmeldung auf und/oder mittels der Vorrichtung mindestens eine erste geschützte Funktion, insbesondere Ausgabe mindestens einer ersten geschützten Information, freizugeben und/oder zu ermöglichen und/oder zu bewirken.

Gelöst wird die Aufgabe auch durch ein System, insbesondere ein erfindungsgemäßes System, welches eingerichtet ist, das erfindungsgemäße Verfahren durchzuführen.

Gelöst wird die Aufgabe auch durch eine Anlage zumindest ein Speichermedium, ein Kommunikationsmittel und einen Prozessor aufweisend, wobei das Speichermedium eine Mehrzahl von zuordenbaren geschützten Funktionen und/oder Informationen beinhaltet, wobei das Kommunikationsmittel zum Empfang eines Autorisierungsvorgangs, insbesondere eines erfindungsgemäßen Autorisierungsvorgangs, und zum Entsenden einer Rückmeldung auf den Autorisierungsvorgang eingerichtet ist, wobei der Prozessor zum Bewirken des mittels der Kommunikationsmittel Sendens der Rückmeldung eingerichtet ist und/oder wobei der Prozessor zum Auswählen einer geschützten Information für die Rückmeldung basierend und/oder abhängig vom Autorisierungsvorgang eingerichtet ist.

Vorteilhafterweise ist die Anlage eingerichtet, das erfindungsgemäße Verfahren auszuführen.

Vorteilhafterweise ist die Anlage eingerichtet, eine Auswahl geschützter Informationen auch bei Ausbleiben sowohl einer bestätigender als auch ablehnender Rückmeldung zu bewirken und diese Auswahl mittels des Kommunikationsmittels zu senden.

Weitere Vorteile und Merkmale der Erfindung ergeben sich aus der nachstehenden Beschreibung eines Ausführungsbeispiels anhand der beigefügten Figuren. Dabei zeigen:
- Fig. 1: eine Darstellung einiger Verfahrensschritte des erfindungsgemäßen Verfahrens und deren mögliche Ausführungsorte,
- Fig. 2: ein Beispiel konkreter Verfahrensschritte des erfindungsgemäßen Verfahrens,
- Fig. 3: eine Darstellung eines möglichen Szenarios unter Zuhilfenahme eines Ausführungsbeispiels des erfindungsgemäßen Systems, erfindungsgemäßer Geräte zur Ausführung des erfindungsgemäßen Verfahrens.

Die Figuren sind exemplarisch und rein schematisch und beschränken sich in ihrer Darstellung auf für das Verständnis der Erfindung wichtigen Merkmale.

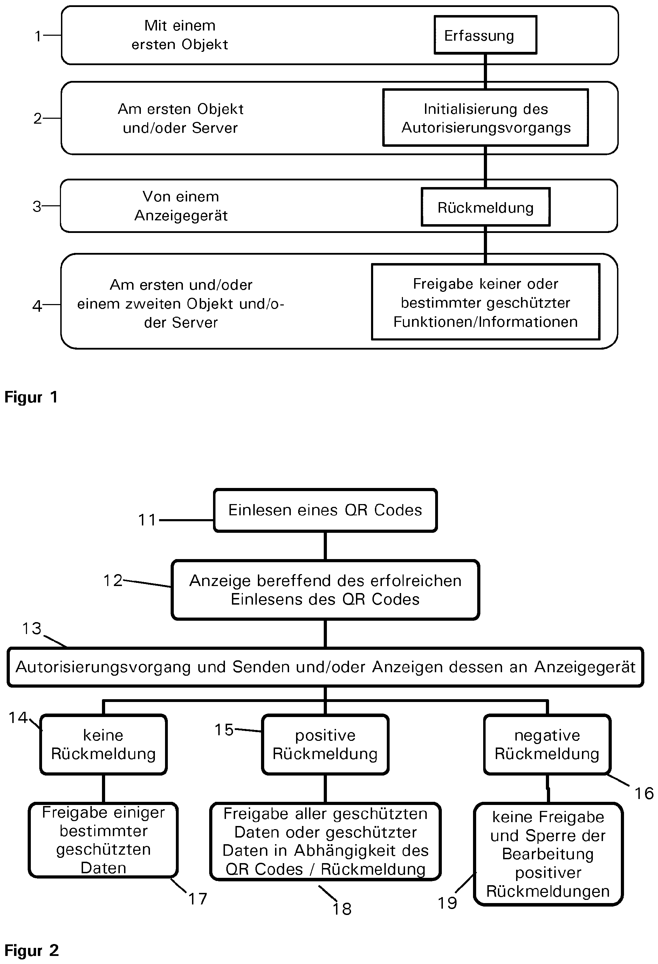

Figur 1 stellt ein Flussdiagramm das erfindungsgemäße Verfahren dar gezeigt, in welchem ebenso mögliche Ausführungsorte bezüglich der einzelnen Schritte des Verfahrens angegeben sind.

In dem erfindungsgemäßen Verfahren wird zunächst im Schritt 1 der Figur 1 eine Erfassung mit einem ersten Objekt durchgeführt. Dieses erste Objekt ist insbesondere ein Erfassungsgerät, wobei mittels des ersten Objekts beispielsweise eine erste Information erfasst und ausgewertet werden kann. Diese Information kann beispielsweise ein QR Code oder Barcode sein, wobei auch eine simple Passworteingabe oder URL Eingabe vorstellbar ist. Diese Information wird insbesondere verglichen, insbesondere mit vorbestimmten Informationen. Aufgrund dieser ersten Erfassung, insbesondere einer aufgrund des Vergleichs der erfassten Information autorisierenden Erfassung, - also durch die erste Erfassung ausgelöst - wird im Schritt 2 der Figur 1 am ersten Objekt ein Autorisierungsvorgang initialisiert. Alternativ wird die Initialisierung des Autorisierungsvorgangs an einem Server bewirkt, wobei das erste Objekt Kommunikationsmittel, vorzugsweise drahtlose Kommunikationsmittel, aufweist, um die Bewirkung der Initialisierung des Autorisierungsvorgangs am Server auszulösen. Die Initialisierung des Autorisierungsvorgangs kann beispielsweise eine Generierung einer Berechtigungsanfrage darstellen oder beinhalten, wobei der Autorisierungsvorgang/die Berechtigungsanfrage zumindest die Möglichkeit einer den Autorisierungsvorgang bestätigenden Rückmeldung oder einer den Autorisierungsvorgang ablehnenden Rückmeldung aufweist. Zusätzlich weist der Autorisierungsvorgang und/oder die Berechtigungsanfrage die Möglichkeit auf mittels eines vorbestimmten berechtigten Eingriffssystems eine Rückmeldung zu generieren. Der Autorisierungsvorgang bzw. die Berechtigungsanfrage kann daraufhin auf einem Anzeigegerät angezeigt werden. Auf diesem Anzeigegerät wird beispielsweise einem Benutzter der Autorisierungsvorgang bzw. dessen mögliche Rückmeldungen angezeigt, sodass dieser die Autorisierung bestätigen bzw. ablehnen kann. Daraufhin wird - wie in Schritt 3 der Figur 1 dargestellt - eine Rückmeldung die Ablehnung bzw. Bestätigung beinhaltend an dem Anzeigegerät generiert und/oder von dem Anzeigegerät aus gesendet. Diese Rückmeldung bewirkt wiederum, wie in Schritt 4 der Figur 1 dargestellt, am ersten Objekt je nach Art der Rückmeldung, also inwiefern eine Bestätigung oder eine Ablehnung, erfolgte, eine oder keine Freigabe geschützter Funktionen/Informationen. Alternativ kann die Bewirkung einer oder keiner Freigabe auch an einem zweiten Objekt oder am Server oder einem weiteren Server erfolgen. Bei einer Ablehnung im Rahmen des Autorisierungsvorgangs wird bevorzugt keine Freigabe einer geschützten Funktion bewirkt, hingegen bei einer Bestätigung im Rahmen des Autorisierungsvorgangs bevorzugt eine Freigabe zumindest eines Teils der geschützten Funktionen. Weiterhin kann das vorbestimmte berechtigte Eingriffssystem bspw. aufgrund eines Einwirkens eines Nutzers, bspw. eines Gesundheitsdienstleisters, eine bestätigende Rückmeldung generieren, beispielsweise mittels der sog. Break-The-Glas Prozedur. Erfolgt keine Rückmeldung, bleibt eine solche demnach aus, so wird dennoch zumindest ein Teil geschützter Funktionen freigegeben. Eine Erweiterung des in diesem Absatz beschriebenen Verfahrens kann dadurch innerhalb des erfindungsgemäßen Verfahrens erreicht werden, dass im Schritt 2 der Figur 1 eine Initialisierung eines Autorisierungsvorgangs durchgeführt, wobei dieser Autorisierungsvorgangs neben der Möglichkeit einer Ablehnung und Bestätigung auch noch die Möglichkeit bietet, eine Auswahl frei zu gebender Funktionen/Informationen, zumindest in vorbestimmten Kategorien, im Autorisierungsvorgang, beispielsweise am Anzeigegerät, anzuzeigen und nach der Rückmeldung vom Anzeigegerät diese Auswahl der geschützten Funktion am ersten und/oder zweiten Objekt und/oder Server und/oder weiteren Server freizugeben.

Das erfindungsgemäße Verfahren lässt sich beispielsweise in dem Ausführungsbeispiel wie in Figur 2 mittels eines Flussdiagramms dargestellt, konkret durchführen. Dabei wird in einem ersten Schritt 11 zunächst eine erste Erfassung dadurch erreicht, dass ein QR-Code eingelesen wird. Dieser QR-Code kann entweder im Zusammenhang mit einer Person stehen, insbesondere durch diese bereitgestellt werden, oder beispielsweise an einem Gerät angebracht sein. Daraufhin erfolgt - wie in Schritt 12 der Figur 2 dargestellt - eine Anzeige des erfolgreichen Erfassens und gegebenenfalls eine Anzeige, dass ein Autorisierungsvorgang aufgrund des erfolgreichen Erfassens initialisiert werden wird. Ein solcher Autorisierungsvorgang, der zumindest eine bestätigende und ablehnenden Rückmeldung erlaubt, wird - wie im Schritt 13 der Figur 2 dargestellt - daraufhin initialisiert und dieser an ein Anzeigegerät gesendet und/oder an einem Anzeigegerät angezeigt. Auf diesem Anzeigegerät kann daraufhin die Autorisierung bestätigt oder diese abgelehnt werden. Bei einer, wie mittels des Schritts 15 in der Figur 2 dargestellten, bestätigenden Rückmeldung erfolgt - wie im Schritt 18 der Figur 2 dargestellt - eine Freigabe aller in der Rückmeldung ausgewählten geschützten Informationen. Bei einer wie im Schritt 16 der Figur 2 gezeigten ablehnen Rückmeldung erfolgt vorzugsweise keine Freigabe einer geschützten Funktion - wie im Schritt 19 der Figur 2 gezeigt. Beispielsweise wird lediglich die Information angezeigt, dass die Autorisierung verweigert wurde. Insbesondere werden bei einer ablehnenden Rückmeldung alle bestätigenden Rückmeldungen und Eingriffe in die Autorisierung ignoriert und können somit nicht zu einer Freigabe geschützter Funktion oder Information führen.

Erfindungswesentlich ist, dass bei einem Ausbleiben einer Rückmeldung, wie im Schritt 14 der Figur 2 gezeigt, trotzdem eine Freigabe einiger vorbestimmter geschützten Daten erfolgt, wie in Schritt 17 der Figur 2 gezeigt. Ein Schweigen auf die Rückmeldung wird demnach nicht als Ablehnung gewertet, sondern ein Teil der Daten wird dennoch ausgegeben.

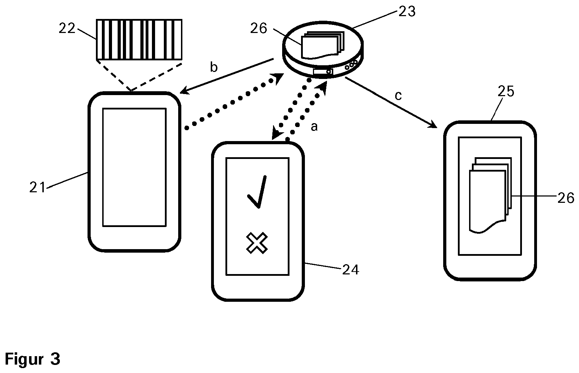

Ein mögliches Beispiel, bei welchem das erfindungsgemäße Verfahren und/oder das erfindungsgemäße System und/oder Anlage Anwendung findet, ist in der Figur 3 skizziert. Die dort dargestellten Geräte 21, 23, 24 und 25 weisen dabei Kommunikationsmittel zur insbesondere drahtlosen Kommunikation auf.

Dabei sind Informationen 26 beispielsweise einer Person auf einem Server 23 gespeichert und geschützt. Diese Personen hat zum Beispiel einen Barcode 22 bei sich, beispielsweise auf einer Karte. Bei einem Unfall dieser Person kann dieser Barcode 22 von einer dritten Person, beispielsweise einem Sanitäter, mittels eines Erfassungsgeräts 21, zum Beispiel einem Smartphone, eingescannt werden. Es ist weiterhin möglich, dass auf dem Erfassungsgerät 21 eine Bestätigungsanzeige des erfolgreichen Erfassens des Barcodes erfolgt. Das Erfassungsgerät 21 sendet die Erfassung des Barcodes 22, also den Umstand einer Erfassung und/oder den Inhalt der Erfassung, an den Server 23. Auf diesem Server 23 wird aufgrund des Empfangs der Erfassung ein Autorisierungsvorgang initialisiert und eine optische Darstellung des Autorisierungsvorgangs, beispielsweise eine Berechtigungsfrage, an ein Anzeigegerät 24 gesendet. Das Anzeigegerät kann beispielsweise ein (weiteres) Smartphone, zum Beispiel eines aufgrund der geschützten Daten ausgewähltes Smartphone der erstgenannten Person, sein. Auf diesem Anzeigegerät 24 wird im Rahmen des Autorisierungsvorgangs dem Benutzer des Anzeigegeräts 24 die Möglichkeit eine den Autorisierungsvorgang bestätigenden und ablehnenden Rückmeldung zu generieren gegeben. Diese Rückmeldung wird, wie mittels des Pfeils a dargestellt, zurück an den Server 23 geleitet.

Möglich ist nun einerseits die Freigabe - dargestellt mittels des Pfeils b - der geschützten Informationen 26 und Anzeige dieser auf dem Erfassungsgerät 21 oder alternativ hierzu - wie dargestellt mittels des Pfeils c - kann eine Freigabe der geschützten Informationen 26 und Anzeige dieser auf einem Ausgabegerät 25 erfolgen. Eine solcher Freigabe erfolgt dann, wenn der Benutzter des Anzeigegeräts 24 eine bestätigende Rückmeldung durch seine Interaktion generiert und diese zum Server 23 gesendet wurde. Die bestätigende Rückmeldung kann in einer vorteilhaften Ausgestaltung auch durch eine Break-The-Glas Prozedur bewirkt werden, beispielsweise durch einen zertifizierten Anbieter. Diese bestätigende Rückmeldung im Rahmen der Break-The-Glass Prozedur kann auch eine beschränkt bestätigende Rückmeldung sein, die zu einer eingeschränkten Freigabe führt, diese Freigabe kann insbesondere umfassender sein als beim Ausbleiben jeglicher bestätigender Rückmeldung.

Ebenso erfolgt eine Freigabe eines Teils der geschützten Funktionen 26, sofern eine Rückmeldung ausbleibt, also beispielsweise keine Rückmeldung auf dem Anzeigegerät 24 generiert wird und an den Server 23 gesendet wird. Auch in einem solchen Fall des "Schweigens", also dem Ausbleiben einer bestätigenden und ablehnenden Rückmeldung ist vorgesehen, dass ein Teil der geschützten Funktionen 26 vom Server 23 freigeben wird und auf dem Erfassungsgerät 21 oder dem Ausgabegerät 25 angezeigt wird.

### Bezugszeichenliste

- 1: Erfassung an einem ersten Objekt, insbesondere einem Erfassungsgerät
- 2: Initialisieren des Autorisierungsvorgangs am ersten Objekt oder Server
- 3: Rückmeldung von einem Anzeigegerät erhalten, beispielsweise am Server
- 4: Freigabe keiner oder bestimmter geschützter Funktionen/Informationen am ersten Objekt oder einem zweiten Objekt oder einem Server
- 11: QR-Code Einlesen
- 12: Anzeige und Statusbericht bzgl. des Einlesens
- 13: Initialisieren des Autorisierungsvorgangs und Anzeigen dessen am Anzeigegerät
- 14: Erfolgen und/oder Erhalten keiner Rückmeldung im Rahmen des Autorisierungsvorgangs
- 15: Erfolgen und/oder Erhalten einer positiven, bestätigender Rückmeldung im Rahmen des Autorisierungsvorgangs
- 16: Erfolgen und/oder Erhalten einer negativen, ablehnender Rückmeldung im Rahmen des Autorisierungsvorgangs
- 17: Freigabe einiger bestimmter geschützter Daten
- 18: Freigabe aller oder einiger Daten
- 19: Keine Freigabe
- 21: Erfassungsgerät
- 22: Barcode
- 23: Server
- 24: Anzeigegerät zur Autorisierung
- 25: Ausgabegerät
- 26: Geschützte Funktionen
- a: Rückmeldung an den Server
- b: Versenden freigegebener, geschützter Informationen an das Erfassungsgerät
- c: Versenden freigegebener, geschützter Informationen an das Ausgabegerät

## Patentansprüche

1. Verfahren zur Freigabe mindestens einer geschützten Funktion, insbesondere Ausgabe mindestens einer geschützten Information, die Schritte beinhaltend:
I. Durchführen einer ersten Erfassung (1),
II. Initialisieren eines von der ersten Erfassung (1) abhängigen und/oder durch die erste Erfassung (1) ausgelösten Autorisierungsvorgangs (2) aufweisend die Möglichkeit zumindest einer bestätigenden und einer ablehnenden Rückmeldung (3) durch einen Benutzer und/oder generiert mittels eines Eingriffssystems,
i. wobei die Rückmeldung (3) im Rahmen des Autorisierungsvorgangs (2) durch den Benutzer erfolgt und/oder eine mittels des Eingriffssystems, insbesondere automatisch, generierte Rückmeldung (3) ist und/oder der Eingriff in den Autorisierungsvorgang (2) und/oder ein Überschreiben des, ein Umgehen des und/oder die Außerkraftsetzung des Autorisierungsvorgangs (2) im Rahmen des Autorisierungsvorgangs (2) aufgrund eines vorbestimmten Protokolls, insbesondere einer Break-The-Glass Prozedur, und/oder mittels eines Nutzers erfolgt,
III. bei Ausbleiben sowohl bestätigender als auch ablehnender Rückmeldung (3, 14) im Rahmen des Autorisierungsvorgangs (2) Bewirken der Freigabe (4) mindestens einer ersten Funktion,
wobei die mindestens eine erste Funktion unabhängig vom Autorisierungsvorgang (2) ist und/oder die mindestens eine erste Funktion eine geschützte Funktion (17, 26) beinhaltet.

2. Verfahren nach Anspruch 1 **dadurch gekennzeichnet, dass** die mindestens eine erste geschützte Funktion (17, 26) mindestens eine erste Information umfasst, wobei insbesondere die mindestens eine erste Information vom Autorisierungsvorgang (2) unabhängige Information beinhaltet und/oder die erste Funktion die Ausgabe einer geschützten Funktion (26) beinhaltet und/oder die Freigabe(4) lediglich einen Teil der geschützten Funktion (17, 26) und/oder lediglich einen Teil der geschützten Informationen beinhaltet.

3. Verfahren nach einem der voranstehenden Ansprüche **dadurch gekennzeichnet, dass** nach einer ablehnenden Rückmeldung im Rahmen des Autorisierungsvorgangs bestätigende Rückmeldungen, insbesondere auf die ablehnende Rückmeldung folgende, im Rahmen des Autorisierungsvorgangs nicht zum Bewirken der Freigabe (4) der mindestens einen ersten und/oder zweiten Funktion führen und/oder nach einer ablehnenden Rückmeldung im Rahmen des Autorisierungsvorgang bestätigende Rückmeldungen im Rahmen des Autorisierungsvorgang ignoriert werden.

4. Verfahren nach einem der voranstehenden Ansprüche **dadurch gekennzeichnet, dass**
bei bestätigender Rückmeldung (15) im Rahmen des Autorisierungsvorgangs (2) die Freigabe mindestens einer zweiten Funktion (18) und/oder Ausgabe mindestens einer zweiten Information bewirkt wird, wobei zumindest ein Teil der mindestens zweiten Funktion und/oder Ausgabe der mindestens zweiten Information eine geschützte Funktion ist und/oder geschützte Informationen beinhaltet, wobei die geschützte Funktion der zweiten Funktion nicht identisch mit der geschützten Funktion der ersten Funktion ist und/oder die geschützte Information der zweiten Information nicht identisch mit der geschützten Informationen der ersten Information ist, und/oder dass
bei einer ablehnenden Rückmeldung (3, 16) im Rahmen des Autorisierungsvorgangs (2) keine Freigabe (19) oder eine Freigabe mit einer dritten Funktion und/oder Ausgabe einer dritten Information bewirkt wird, wobei insbesondere die dritte Funktion keine geschützte Funktion ist und/oder insbesondere die dritte Information keine geschützte Information beinhaltet.

5. Verfahren nach dem voranstehenden Anspruch **dadurch gekennzeichnet, dass** die dritte Funktion eine von und/oder auf Parametern und/oder Inhalt der Rückmeldung im Rahmen des Autorisierungsvorgangs abhängige und/oder basierende geschützte Funktion ist, wobei die geschützte Funktion der dritten Funktion nicht identisch mit der geschützten ersten und/oder zweiten Funktion ist.

6. Verfahren nach einem der voranstehenden Ansprüche **dadurch gekennzeichnet, dass** es den Schritt einer Initialfreigabe umfasst.

7. Verfahren nach dem voranstehenden Anspruch **dadurch gekennzeichnet, dass** die Initialfreigabe mindestens eine Initialfunktion und/oder eine Initialausgabe mit mindestens einer Initialinformation umfasst.

8. Verfahren nach dem voranstehenden Anspruch **dadurch gekennzeichnet, dass** die Initialinformation und/oder Initialfunktion lediglich einen Teil der geschützten Information und/oder Funktion beinhaltet.

9. Verfahren nach einem der voranstehenden Ansprüche **dadurch gekennzeichnet, dass** der Autorisierungsvorgang durch und/oder abhängig von der/die erste(n) Erfassung und/oder der/die Initialausgabe und/oder der/die Initialfreigabe und/oder vorbestimmten und/oder gespeicherten Daten und/oder Konfiguration initialisiert wird.

10. Verfahren nach einem der voranstehenden Ansprüche **dadurch gekennzeichnet, dass** die Initialisierung (2) zur Benutzerinteraktion und/oder an ein und/oder hin zu einem tragbare(s/n) und/oder mobile(s/n) und/oder mitführbare(s/n) und/oder persönliche(s/n) Gerät bewirkt wird.

11. Verfahren nach einem der voranstehenden Ansprüche **dadurch gekennzeichnet, dass** das Ausbleiben einer Rückmeldung (14) ein Ausbleiben einer Rückmeldung innerhalb eines vorbestimmten Zeitintervalls ist.

12. Verfahren nach einem der voranstehenden Ansprüche **dadurch gekennzeichnet, dass** bei einer Rückmeldung im Rahmen des Autorisierungsvorgangs (2) die Rückmeldung mindestens einen Parameter beinhaltet, wobei mindestens die erste und/oder zweite und/oder dritte Funktion und/oder Information aufgrund des Parameters ausgewählt wird.

13. Verfahren nach einem der voranstehenden Ansprüche, wobei die Freigabe der mindestens einen ersten Funktion und/oder Ausgabe der mindestens einen ersten Information und/oder der mindestens einen zweiten Funktion und/oder Ausgabe der mindestens einen zweiten Information und/oder der mindestens einen dritte Funktion und/oder Ausgabe der mindestens einen dritten Information und/oder der mindestens einen vierten Funktion und/oder Ausgabe der mindestens einen vierten Information und/oder die Initialfreigabe der Initialfunktion und/oder Initialausgabe der mindestens einen Initialinformation eine Anzeige und/oder Ausführung der ersten, zweiten, dritten und/oder vierten Funktion und/oder Information und/oder Initialfunktion und/oder Initialinformation bewirkt und/oder umfasst.

14. System zur kontrollierten Freigabe mindestens einer geschützten Funktion, insbesondere ausgebend mindestens eine geschützte Information, aufweisend
ein Eingabegerät (21) zum Erfassen einer ersten Anfrage,
ein Kommunikationsmittel zum Senden eines Autorisierungsvorgangs zum zumindest Bestätigen oder Ablehnen der ersten Anfrage,
wobei das Bestätigen oder Ablehnen im Rahmen des Autorisierungsvorgangs (2) durch den Benutzer erfolgt und/oder eine mittels des Eingriffssystems, insbesondere automatisch, generierte Bestätigung oder Ablehnung ist und/oder der Eingriff in den Autorisierungsvorgang (2) und/oder ein Überschreiben des, ein Umgehen des und/oder die Außerkraftsetzung des Autorisierungsvorgangs (2) im Rahmen des Autorisierungsvorgangs (2) aufgrund eines vorbestimmten Protokolls, insbesondere einer Break-The-Glass Prozedur, und/oder mittels eines Nutzers erfolgt,
ein Empfangsmittel zum Empfangen einer Rückmeldung über den Autorisierungsvorgang, insbesondere dessen Bestätigung oder Ablehnung, eine Vorrichtung aufweisend und/oder eingerichtet zum Ausführen geschützter Funktionen, insbesondere ein Ausgabegerät zur Anzeige geschützter Information, und
ein Freigabemittel zur Freigabe der geschützten Funktionen auf und/oder mittels der Vorrichtung, insbesondere Ausgabe der geschützten Information auf und/der mittels dem/des Ausgabegerät(s),
wobei das Freigabemittel eingerichtet ist, bei und nach dem Erfassen der ersten Anfrage und Ausbleiben eines Empfangs einer, insbesondere sowohl bestätigender als auch ablehnender, Rückmeldung auf und/oder mittels der Vorrichtung mindestens eine erste geschützte Funktion freizugeben (17), insbesondere eine Ausgabe mindestens einer ersten geschützten Information freizugeben.

## Claims

1. A method for enabling at least one protected function, in particular output of at least one protected information, comprising the steps:
I. carrying out a first acquisition (1),
II. initialising an authorisation process (2) dependent on the first detection (1) and/or triggered by the first detection (1), comprising the possibility of at least one confirming and one rejecting feedback (3) by a user and/or generated by means of an intervention system,
1. wherein the feedback (3) within the scope of the authorisation procedure (2) is effected by the user and/or is a feedback (3) generated by means of the intervention system, in particular automatically, and/or the intervention in the authorisation procedure (2) and/or an overwriting of the authorisation procedure (2), a bypassing of the authorisation procedure (2) and/or the overriding of the authorisation procedure (2) within the scope of the authorisation procedure (2) is effected on the basis of a predetermined protocol, in particular a break-the-glass procedure, and/or by means of a user,
III. in the absence of both confirming and rejecting feedback (3, 14) during the authorisation procedure (2), effecting the release (4) of at least one first function,
wherein said at least one first function is independent of the authorisation process (2) and/or said at least one first function includes a protected function (17, 26).

2. A method according to claim 1, **characterised in that** said at least one first protected function (17, 26) comprises at least one first information, wherein in particular said at least one first information comprises information independent of the authorisation process (2) and/or the first function comprises the output of a protected function (26)
and/or the release (4) contains only a part of the protected function (1 7, 26) and/or only a part of the protected information.

3. A method according to one of the preceding claims, **characterised in that** after a rejecting feedback within the scope of the authorisation process, confirming feedbacks, in particular feedbacks following the rejecting feedback, within the scope of the authorisation process do not lead to the release (4) of said at least one first and/or second function being effected and/or after a rejecting feedback within the scope of the authorisation process, confirming feedbacks within the scope of the authorisation process are ignored.

4. A method according to one of the preceding claims, **characterised in that**
in the event of confirming feedback (15) in the context of the authorisation process (2), the release of at least one second function (18) and/or output of at least one second item of information is effected, at least part of said at least second function and/or output of said at least second item of information being a protected function and/or containing protected information, the protected function of the second function not being identical to the protected function of the first function and/or the protected information of the second item of information not being identical to the protected information of the first item of information, and/or **in that**
in the event of a negative response (3, 16) in the context of the authorisation procedure (2), no release (19) or a release with a third function and/or output of a third information is effected, wherein in particular the third function is not a protected function and/or in particular the third information does not contain any protected information.

5. A method according to the preceding claim, **characterised in that** the third function is a protected function dependent on and/or based on parameters and/or content of the feedback in the context of the authorisation process, wherein the protected function of the third function is not identical to the protected first and/or second function.

6. A method according to one of the preceding claims, **characterised in that** it comprises the step of an initial release.

7. A method according to the preceding claim, **characterised in that** the initial release comprises at least one initial function and/or an initial output with at least one initial information.

8. A method according to the preceding claim, **characterised in that** the initial information and/or initial function contains only a part of the protected information and/or function.

9. A method according to any one of the preceding claims, **characterised in that** the authorisation process is initialised by and/or dependent on the initial capture(s) and/or initial output(s) and/or initial release(s) and/or predetermined and/or stored data and/or configuration.

10. A method according to any one of the preceding claims, **characterised in that** the initialisation (2) is effected for user interaction and/or to and/or towards one / several portable and/or mobile and/or carryable and/or personal devices.

11. A method according to any one of the preceding claims, **characterised in that** the absence of feedback (14) is an absence of feedback within a predetermined time interval.

12. A method according to one of the preceding claims, **characterised in that**, in the case of a feedback in the context of the authorisation process (2), the feedback includes at least one parameter, at least the first and/or second and/or third function and/or information being selected on the basis of the parameter.

13. A method according to one of the preceding claims, wherein the release of said at least one first function and/or output of said at least one first information and/or said at least one second function and/or output of said at least one second information and/or said at least one third function and/or output of said at least one third information and/or said at least one fourth function and/or output of said at least one fourth information and/or the initial release of the initial function and/or initial output of said at least one initial information causes and/or comprises a display and/or execution of the first, second, third and/or fourth function and/or information and/or initial function and/or initial information.

14. A system for the controlled release of at least one protected function, in particular comprising at least one piece of protected information, comprising
an input device (21) for recording a first request, a communication means for sending an authorisation process for at least confirming or rejecting the first request, wherein the confirmation or rejection is carried out by the user within the scope of the authorisation process (2) and/or is a confirmation or rejection generated by means of the intervention system, in particular automatically, generated by means of the intervention system, in particular automatically, and/or the intervention in the authorisation process (2) and/or an overwriting of the authorisation process (2), a bypassing of the authorisation process (2) and/or the overriding of the authorisation process (2) within the framework of the authorisation process (2) takes place on the basis of a predetermined protocol, in particular a break-the-glass procedure, and/or by means of a user,
a receiving means for receiving a feedback about the authorisation process, in particular its confirmation or rejection, a device comprising and/or arranged for executing protected functions, in particular an output device for displaying protected information, and
an enabling means for enabling the protected functions on and/or by means of the device, in particular output of the protected information on and/or by means of the output device(s),
wherein the release means is set up to release (17) at least one first protected function, in particular to release an output of at least one first protected item of information , upon and after the detection of the first request and in the absence of a receipt of a response, in particular both confirming and rejecting, on and/or by means of the device.

## Revendications

1. Procédé pour activer au moins une fonction protégée, en particulier la sortie d'au moins une information protégée, comprenant les étapes suivantes :
I. effectuer une première acquisition (1),
II. initialiser un processus d'autorisation (2) dépendant de la première détection (1) et/ou déclenché par la première détection (1), comprenant la possibilité d'au moins une rétroaction de confirmation et une rétroaction de rejet (3) par un utilisateur et/ou générée au moyen d'un système d'intervention,
1. dans lequel le retour d'information (3) dans le cadre de la procédure d'autorisation (2) est effectué par l'utilisateur et/ou est un retour d'information (3) généré au moyen du système d'intervention, en particulier automatiquement, et/ou l'intervention dans la procédure d'autorisation (2) et/ou un écrasement de la procédure d'autorisation (2), un contournement de la procédure d'autorisation (2) et/ou le remplacement de la procédure d'autorisation (2) dans le cadre de la procédure d'autorisation (2) est effectué sur la base d'un protocole prédéterminé, en particulier une procédure de bris de glace, et/ou au moyen d'un utilisateur,
III. en l'absence d'un retour d'information de confirmation et de rejet (3, 14) pendant la procédure d'autorisation (2), effectuer la libération (4) d'au moins une première fonction,
dans lequel ladite au moins une première fonction est indépendante du processus d'autorisation (2) et/ou ladite au moins une première fonction comprend une fonction protégée (17, 26).

2. Procédé selon la revendication 1, **caractérisé en ce que** ladite au moins une première fonction protégée (17, 26) comprend au moins une première information, dans lequel en particulier ladite au moins une première information comprend une information indépendante du processus d'autorisation (2) et/ou la première fonction comprend la sortie d'une fonction protégée (26).
et/ou le communiqué (4) ne contient qu'une partie de la fonction protégée (1 7, 26) et/ou qu'une partie des informations protégées.

3. Procédé selon l'une des revendications précédentes, **caractérisé en ce qu'**après un retour d'information de rejet dans le cadre du processus d'autorisation, les retours d'information de confirmation, en particulier les retours d'information suivant le retour d'information de rejet, dans le cadre du processus d'autorisation ne conduisent pas à la libération (4) de ladite au moins une première et/ou deuxième fonction et/ou après un retour d'information de rejet dans le cadre du processus d'autorisation, les retours d'information de confirmation dans le cadre du processus d'autorisation sont ignorés.

4. Procédé selon l'une des revendications précédentes, **caractérisé en ce que**
en cas de confirmation du retour d'information (15) dans le cadre du processus d'autorisation (2), la libération d'au moins une deuxième fonction (18) et/ou la sortie d'au moins un deuxième élément d'information est effectuée, au moins une partie de ladite au moins deuxième fonction et/ou de la sortie dudit au moins deuxième élément d'information étant une fonction protégée et/ou contenant des informations protégées, la fonction protégée de la deuxième fonction n'étant pas identique à la fonction protégée de la première fonction et/ou les informations protégées du deuxième élément d'information n'étant pas identiques aux informations protégées du premier élément d'information, et/ou **en ce que**
en cas de réponse négative (3, 16) dans le cadre de la procédure d'autorisation (2), aucune libération (19) ou une libération avec une troisième fonction et/ou une sortie d'une troisième information est effectuée, la troisième fonction n'étant notamment pas une fonction protégée et/ou la troisième information ne contenant notamment aucune information protégée.

5. Procédé selon la revendication précédente, **caractérisé en ce que** la troisième fonction est une fonction protégée dépendant de et/ou fondée sur des paramètres et/ou le contenu du retour d'information dans le contexte du processus d'autorisation, dans lequel la fonction protégée de la troisième fonction n'est pas identique à la première et/ou la deuxième fonction protégée.

6. Procédé selon l'une des revendications précédentes, **caractérisé en ce qu'**il comprend l'étape d'une libération initiale.

7. Procédé selon la revendication précédente, **caractérisé en ce que** la libération initiale comprend au moins une fonction initiale et/ou une sortie initiale avec au moins une information initiale.

8. Procédé selon la revendication précédente, **caractérisé en ce que** l'information initiale et/ou la fonction initiale ne contient qu'une partie de l'information et/ou de la fonction protégée.

9. Procédé selon l'une quelconque des revendications précédentes, **caractérisé en ce que** le processus d'autorisation est initialisé par et/ou dépendant de la ou des captures initiales et/ou de la ou des sorties initiales et/ou de la ou des libérations initiales et/ou des données et/ou de la configuration prédéterminées et/ou stockées.

10. Procédé selon l'une quelconque des revendications précédentes, **caractérisé en ce que** l'initialisation (2) est effectuée pour une interaction de l'utilisateur et/ou vers et/ou en direction d'un / plusieurs dispositifs portables et/ou mobiles et/ou transportables et/ou personnels.

11. Procédé selon l'une quelconque des revendications précédentes, **caractérisé en ce que** l'absence de retour (14) est une absence de retour dans un intervalle de temps prédéterminé.

12. Procédé selon l'une des revendications précédentes, **caractérisé en ce que**, dans le cas d'un retour d'information dans le cadre du processus d'autorisation (2), le retour d'information comprend au moins un paramètre, au moins la première et/ou la deuxième et/ou la troisième fonction et/ou information étant sélectionnée en fonction du paramètre.

13. Procédé selon l'une des revendications précédentes, dans lequel la libération de ladite au moins une première fonction et/ou sortie de ladite au moins une première information et/ou de ladite au moins une deuxième fonction et/ou sortie de ladite au moins une deuxième information et/ou de ladite au moins une troisième fonction et/ou sortie de ladite au moins une troisième information et/ou de ladite au moins une quatrième fonction et/ou sortie de ladite au moins une quatrième information et/ou la libération initiale de la fonction initiale et/ou sortie initiale de ladite au moins une information initiale provoque et/ou comprend un affichage et/ou une exécution de la première, deuxième, troisième et/ou quatrième fonction et/ou information et/ou fonction initiale et/ou information initiale.

14. Système pour la libération contrôlée d'au moins une fonction protégée, comprenant en particulier au moins une information protégée, comprenant
un dispositif d'entrée (21) pour l'enregistrement d'une première demande, un moyen de communication pour l'envoi d'un processus d'autorisation pour au moins la confirmation ou le rejet de la première demande, la confirmation ou le rejet étant effectué par l'utilisateur dans le cadre du processus d'autorisation (2) et/ou étant une confirmation ou un rejet généré au moyen du système d'intervention, en particulier automatiquement, généré au moyen du système d'intervention, en particulier automatiquement, et/ou l'intervention dans le processus d'autorisation (2) et/ou un écrasement du processus d'autorisation (2), un contournement du processus d'autorisation (2) et/ou le remplacement du processus d'autorisation (2) dans le cadre du processus d'autorisation (2) a lieu sur la base d'un protocole prédéterminé, en particulier une procédure de bris de glace, et/ou par l'intermédiaire d'un utilisateur,
un moyen de réception pour recevoir un retour d'information sur le processus d'autorisation, en particulier sa confirmation ou son rejet, un dispositif comprenant et/ou agencé pour exécuter des fonctions protégées, en particulier un dispositif de sortie pour afficher des informations protégées, et
un moyen de validation pour valider les fonctions protégées sur et/ou au moyen du dispositif, en particulier la sortie des informations protégées sur et/ou au moyen du ou des dispositifs de sortie,
dans lequel le moyen de libération est configuré pour libérer (17) au moins une première fonction protégée, en particulier pour libérer une sortie d'au moins un premier élément d'information protégé, sur et après la détection de la première demande et en l'absence d'une réception d'une réponse, en particulier à la fois de confirmation et de rejet, sur et/ou au moyen du dispositif.
